# EUROPEAN PATENT SPECIFICATION

(11) **EP 2 120 760 B1**
(45) Date of publication and mention of the grant of the patent: **16.09.2015**
(21) Application number: 08713972.1
(22) Date of filing: 24.01.2008
(51) Int. Cl.: A61B 18/22

(54) **MODULAR LASER SYSTEMS**
MODULARE LASERSYSTEME
SYSTÈMES LASER MODULAIRES

(30) Priority: 21.02.2007 US 891037 P
(43) Date of publication of application: 25.11.2009
(73) Proprietor: Cao Group, Inc., West Jordan, Utah 84084 (US)
(72) Inventor: CAO, Densen, Sandy, Utah 84093 (US)
(74) Representative: Leppard, Andrew John
(86) International application number: PCT/US2008/051940
(87) International publication number: WO 2008/103519

(56) References cited:
- WO-A1-03/103529
- WO-A2-2004/091380
- US-A- 4 632 505
- US-A- 5 825 958
- US-A1- 2006 064 080
- US-A1- 2006 095 095

## Description

### TECHNICAL FIELD

The present invention relates to the field of surgical and therapeutic devices and more particularly relates to the field of laser surgical and therapeutic devices.

### BACKGROUND ART

Surgical and therapeutic lasers using semiconductor laser as light source have been widely used in the medicine, dentistry and other areas. In order to increase the usage by practitioners, features of laser system need to be improved. A surgical laser with a fiber management system and disposable tips was described in WO2008/103859. The present invention, an improvement over the aforementioned, utilizes a modular system with wireless control, touch screen programming, a removable fiber cable, autoclaveable hand piece, and versatile surgical tips.

US 2006/0064080 A1 discloses a laser system that includes a removable fibre module that manages fibre dispensing to avoid damage to or waste of fibre. A hand piece is provided. The hand piece includes a channel to guide fibre from a fibre cartridge through the hand piece. There is a stopper in the rear end of the hand piece to hold the fibre steady. On the other side of the hand piece, there is a connector to fit a laser tip. The tip is removable from the hand piece.

US 4,632,505 discloses an optical fibre connector. A tubular body is provided which engages with another tubular body.

WO 03/103529 discloses a medical tool for dental treatments. A handpiece is provided which fits into a rear part.

US 5,825,958 discloses a hand held laser device which includes a handpiece coupled to a source of laser energy by an optical fibre cable.

US 2006/0095095 discloses a method of killing cancer cells which includes placing fibre needles into a human body adjacent cancerous cells and exposing the cancerous cells to laser light emitted through the fibre needles so that the laser light tends to cause death of the cancer cells. The fibre needles comprise a fibre connector and abutment for coupling with end connectors. The fibre needle has a stem and a channel housing a fiber.

### DISCLOSURE OF THE INVENTION

In view of the foregoing disadvantages inherent in the known types of laser systems, an aspect of the present invention provides a disposable laser transmission tip as claimed in claim 1. Embodiments provide an improved laser system with a laser module capable to provide multiple wavelengths, wireless remote control, an improved fiber optic coupling system for laser delivery, auto cleavable handpiece, replaceable tip structure. As such, embodiments of the present invention's general purpose is to provide a new and improved laser system that is effective in use and easy and intuitive in that use.

To accomplish these objectives, the laser system according to the invention is practiced in two embodiments, both of which comprise a control module and a remote foot pedal operation control. In a first embodiment, the control module is a battery powered remote module which is easily maneuverable to a desired location. In the second, the control module is a relatively fixed consol and a separate handpiece is instead battery powered and movable. Both embodiments feature a laser module with multiple wavelength emission capability, a touch screen consol, a new fiber coupling system and replaceable therapeutic/surgical tips.

The more important features of the invention have thus been outlined in order that the more detailed description that follows may be better understood and in order that the present contribution to the art may better be appreciated. Additional features of the invention will be described hereinafter and will form the subject matter of the claims that follow.

Many objects of this invention will appear from the following description and appended claims, reference being made to the accompanying drawings forming a part of this specification wherein like reference characters designate corresponding parts in the several views.

Before explaining at least one embodiment of the invention in detail, it is to be understood that the invention is not limited in its application to the details of construction and the arrangements of the components set forth in the following description or illustrated in the drawings. The invention is capable of other embodiments and of being practiced and carried out in various ways. Also it is to be understood that the phraseology and terminology employed herein are for the purpose of description and should not be regarded as limiting.

### BRIEF DESCRIPTION OF THE DRAWINGS

Figure 1 is a plan view of the first embodiment of the surgical laser system according to the present invention.
Figure 2 is a plan view of a second embodiment of the invention, utilizing a wireless handpiece.
Figure 3 depicts electronic architect of modular laser system illustrated in Figure 1.
Figure 4 depicts electronic architect of modular laser system illustrated in Figure 2.
Figure 5 is a schematic depicting a laser module to provide multiple wavelengths for the laser system.
Figure 6(a) is a schematic depicting one of laser beam delivery mechanism designed for laser system.
Figure 6(b) depicts a coupler housing
Figure 6(c) depicts the assembled laser beam delivery in Figure 6a
Figure 6(d) depicts the optical beam trace mechanism for laser beam delivery described in Fig. 6(a).
Figure 6(e) depicts a different laser beam delivery mechanism for designed laser system.
Figure 6(f) depicts the assembled laser beam deliver in Figure 6e.
Figure 6(g) depicts the optical beam trace mechanism for laser beam delivery described in Figure 6e.
Figure 6(h) depicts another laser beam delivery system
Figure 6(i) depicts assembled laser beam delivery described in Figure 6h.
Figure 6(j) depicts the optical beam trace mechanism for laser beam delivery described in Figure 6h.
Figures 7(a) and 7(b) are schematics depicting alternate laser tips for the present invention.
Figures 8(a) - 8(e) depict sample tips, of the design shown in FIG. 7b, set at different angles.

### MODES FOR CARRING OUT THE INVENTION

With reference now to the drawings, the preferred embodiment of the improved prophy cup is herein described. It should be noted that the articles "a", "an" and "the", as used in this specification, include plural referents unless the content clearly dictates otherwise.

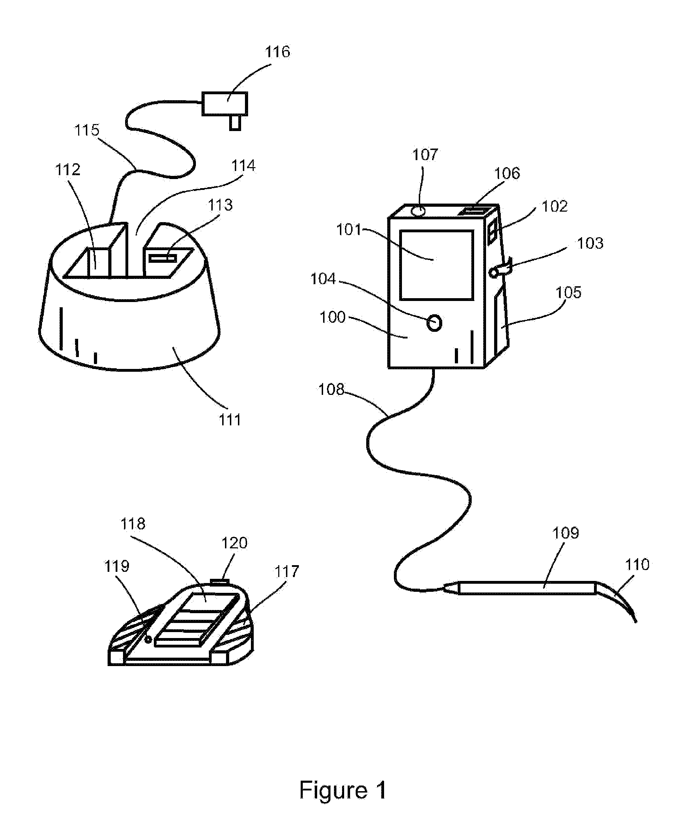

Figure 1 depicts a modular system laser with a main consol and a wireless footswitch where control consol 100 has a touch screen 101, a main electrical switch 102, a handpiece holder 103, an emergency stop button 104, a battery pack 105 to make the unit operable by battery, a USB port 106 to update system operating software, a remote control port 107 to control laser emission remotely if needed, a fiber cable 108 extending from control consol 100, a handpiece 109 connected to fiber cable 108 generally opposite the consol 100 and a disposable tip 110 connected to handpiece 109. The preferred embodiment of the system as a whole likewise comprises a cradle 111 to house the control consol 100. The cradle 111 has an open slot 112 for consol 100 to sit. A connection pin 113 is disposed within the slot 112 to connect electrical power from cradle 111 to control consol 100. There is a secondary slot 114 to allow fiber cable in the consol 100 to go through cradle 111 when the consol 100 sits in the cradle. An electrical cord 115, with an appropriate power supply 116, is connected to the cradle 111 and is in operable connection to the connector pin 113. The electrical power supply 116 and 115 can also connect to consol 100 directly without a cradle. The preferred embodiment of the system also comprises a wireless footswitch 117 to control the laser emission. The wireless footswitch contains a footswitch 118, a multiple color LED indicator 119 for battery and signal status, and a reset button 120.

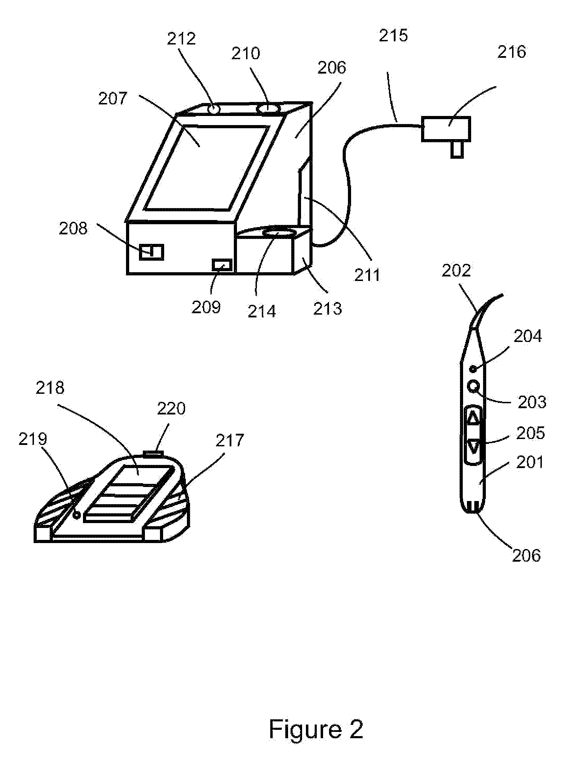

In FIG. 2, the laser system has a wireless laser handpiece 201 with a disposable tip 202. The handpiece 201 is battery operated. Handpiece 201 also features an emergency stop button 203 and a laser emission indicator 204. There is also a laser intensity adjustment control 205 on the laser handpiece 201. Like the previous embodiment, the system contains a control consol 206 with a touch screen 207, a main power switch 208, a USB port 209 for programming updates, an emergency stop button 210, a battery pack 211 and a remote control switch 212. In this embodiment, the consol 206 also comprises a hand piece holder 213, an open slot 214 in 213 for handpiece to sit, a removable electrical cable 215 attached to control consol 206 for charging purposes (actual connection means between the cable 215 and the open slot 214, for charging the handpiece 201, is not shown), and a switch power supply 216 to provide electrical power. The system also include a wireless footswitch 217 including a main footswitch 218, a multiple color LED indicator 219 for battery and signal status and a reset button 220.

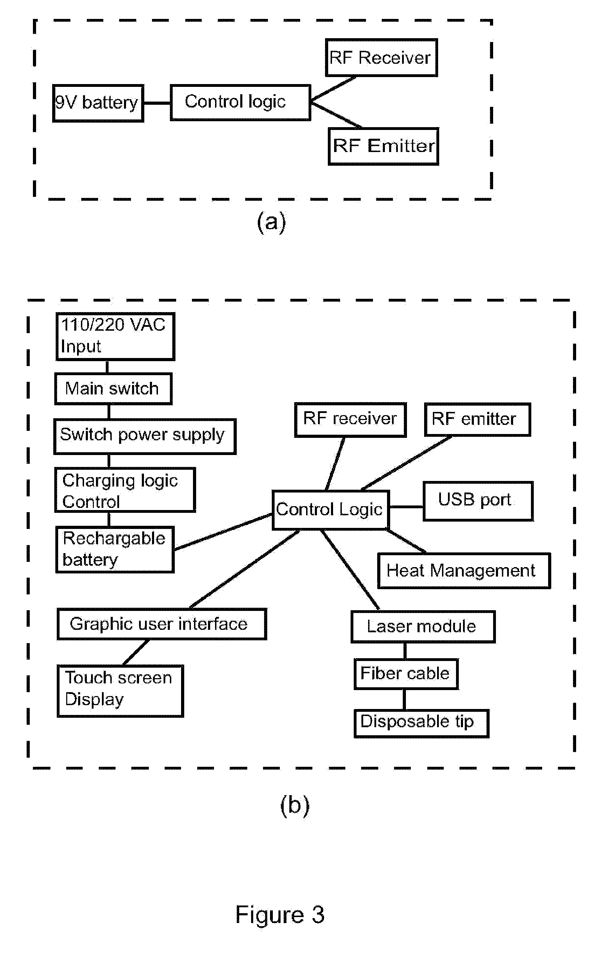

Figure 3 depicts the electrical architecture of the first embodiment where block (a) contains electrical design for wireless footswitch. The footswitch is powered by battery and is operated by a control logic circuit which process signals for an electronic signal emitter and receiver (denoted as ES receiver and ES emitter in the Figures). It should be noted that, as used in this Application, the term "electronic signal" includes any means of wireless communication now known or later developed, including but not limited to Laser, IR, RF, and BLUETOOTH communications. Block (b) illustrates the architectural design for main control. There is a battery charging section as the unit is operated by battery. The signal is processed through control logic circuit. The information is input by touch screen through a graphic user interface. The signal from foot switch controls laser emission by sending electronic signals to the system as a whole. The control program can be updated through a USB port.

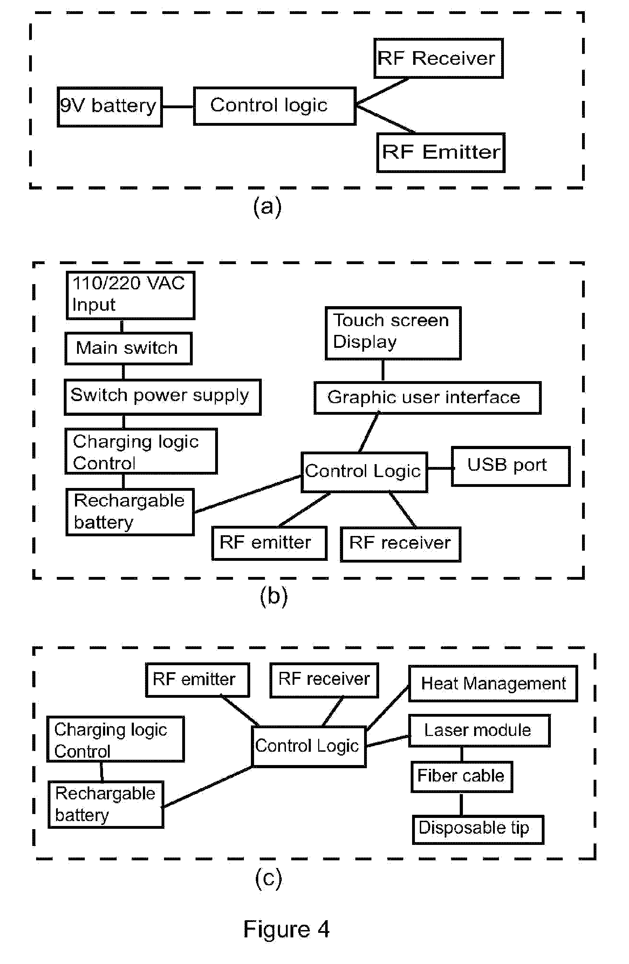

Similarly in FIG. 4, where the architecture is for the system in FIG. 2, block (a) illustrates electrical design for wireless footswitch. The footswitch is powered by battery to operate a control logic which process signals for the electronic signal emitter and receivers. Block (b) illustrates the architectural design for main control. There is a battery charging section as the control console and handpiece are operated by battery. The signal is processed through a control logic circuit. The information is inputted by touch screen through a graphic user interface. The control program can be updated by a USB port. Block (c) illustrates the architect design for a laser handpiece which is operated by battery. There is an electronic signal emitter and receiver in the handpiece to receive/send signals from and to main control unit. The information is processed by control logic circuit to control laser emission. The laser emission is controlled by wireless signal from footswitch.

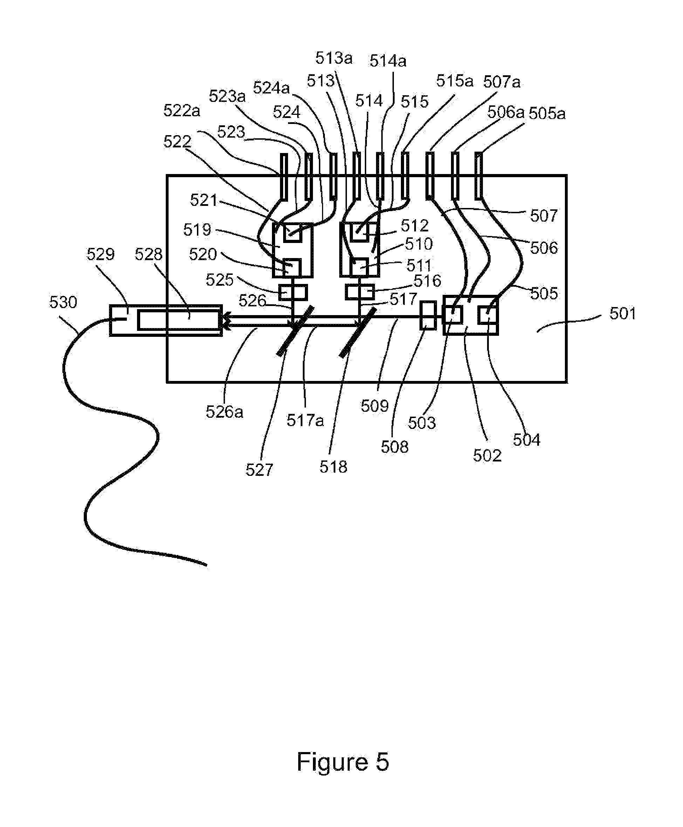

Both embodiments use a laser module to generate a multiple wavelength laser beam for emission through a single fiber. It should be noted that the laser module is located in the consol in the first embodiment (FIG. 3) and the handpiece in the second (FIG. 4). Figure 5 depicts a laser module used in both embodiments. The laser module depicted in Fig.5 can be either a laser module capable of emitting a single wavelength or multiple wavelengths, dependent upon the types of laser chips used in the module. The laser module is encased in a metal housing 501. Inside housing 501, a heat sink 502 carries a laser chip 503 and a detector chip 504. The detector chip 504 detects the laser signal so that the emission of laser power can be controlled. The laser chip 503 and detector chip 504 are bonded by conduction wires 505, 506, 507 respectively to the electrodes 505a, 506a, and 507a on the housing 501, respectively. In front of laser chip 504, there is an optical lens 508 to make the emitted laser beam become a parallel beam 509 for transport.

Another heat sink 510 carries a laser chip 511 and a detector chip 512. The laser chip and detector chips are bonded by conduction wires 513, 514, and 51 5 to the electrodes 51 3a, 514a, and 515a respectively. There is an optical lens 516 to make the emitted laser beam become a parallel beam 51 7. Both beam 509 and 51 7 meet with a filter/reflector 518 which is 100% transparent to beam 509 and 100% reflective to beam 51 7, reflecting beam 51 7 to make create beam 51 7a. The reflectivity and transparency of this filter/reflector 518 is due to one side of the filter/reflector 518 being transparent to all or at least most wavelengths of laser light while the other is reflective of all or most wavelengths of laser light.

Yet another heat sink 519 carries laser chip 520 and detector chip 521. The laser chip and detector chips are bonded by conductive wires 522, 523, and 524 to the electrodes 522a, 523a, and 524a respectively. There is an optical lens 525 to make the emitted laser beam become a parallel beam 526. Beam 526, 509, 51 7a meet with a filer/reflector 527 which are 100% transparent to 509 and 51 7a and 100% reflective to beam 526, reflecting bean 526 to create beam 526a. All three beams, 509, 51 7a, 526a reach an optical lens 528 housed by holder 529. Lens 528 focuses all three beams into a single fiber 530. Thus, with three generated laser beams merged into a single beam, the fiber can emit a single laser beam with three different wavelengths. It is conceivable that additional laser sources may be used to add more wavelengths to the final emitted beam.

Delivering a laser beam to a surgical surface is a key for the laser system. Several laser beam delivery mechanisms will be disclosed herein.

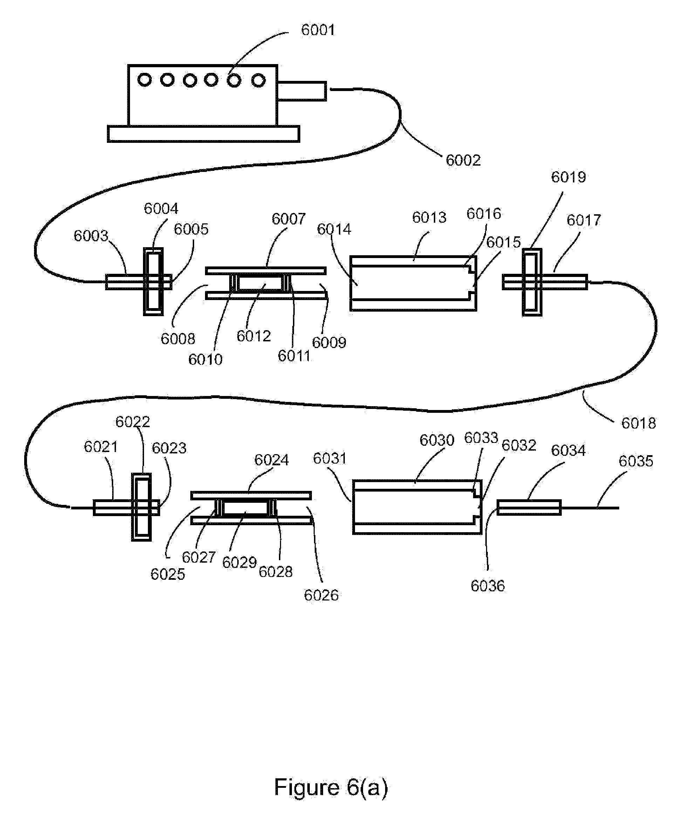

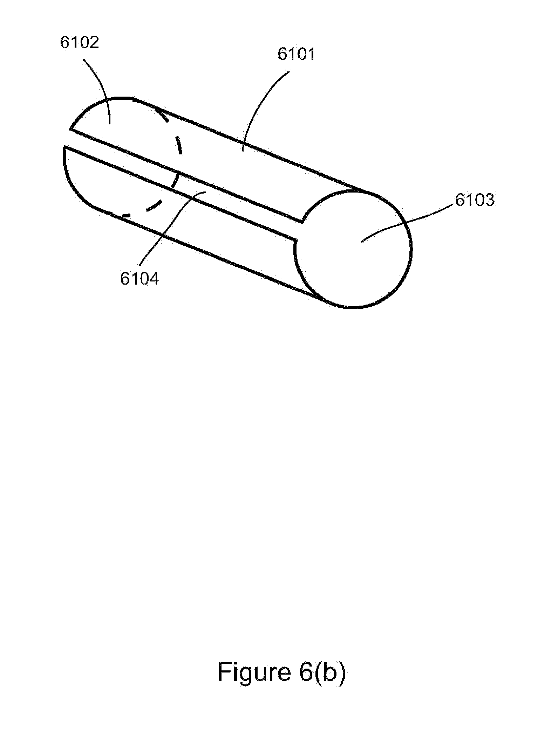

Fig. 6(a) describes one of the delivery mechanisms for a laser beam. Given a laser module 6001 as described in Figure 5, the system according to the present invention is then assembled with the laser module 6001 as a centerpiece, shown in FIG. 6(a). Fiber 6002 exits module 6001 to connect to other components. A ferrule 6003 is provided to the fiber 6002 so as to connect the fiber 6002 to the next stage. A nut 6004 connected to ferrule 6003 facilitates the connection of ferrule 6003 to other connections. The fiber 6002 is finished at end of ferrule 6005 with standard fiber finish. Then, there is a housing 6007 with an opening 6008 at proximal end and another opening 6009 at distal end. There are precision spacers 6010 and 6011 at both ends of an optical lens 6012, inside housing 6007. The details for housing 6007 will be described in Fig. 6(b). A coupler 6013 is provided for further light transportation. The coupler 6013 with opening 6014 at proximal end opening 6015 at distal end, and a stop point 6016 contains housing 6007. Then, a ferrule 6017 contains another fiber 6018. A nut 6019 is connected to 6017 for attachment. Fiber 6018 has a standard finish 6020 for at end 6017. At another end of fiber 6018, there is a ferrule 6021 to make fiber to connect to next stage. A nut 6022 is attached to ferrule 6021 and a fiber finish surface 6023 at end of ferrule 6020. Another housing 6024 with opening 6025 at proximal end and opening 6026 at distal end contains a precision spacer 6027 and 6028 at both end of an optical lens 6028, respectively. A coupler 6030 with opening 6031 at proximal end, opening 6032 at distal end, and a stop point 6033 to house 6024. Another ferrule 6034 to contain fiber 6035 with fiber finish 6036 can be fit to coupler 6030.

Fig. 6(b) depicts details the housing 6007 and 6024. A cylindrical housing 6101 which can be made of metal or plastic with elastic properties has an opening 6102 at proximal end, opening 6103 at distal end, and an open slot 6104 from proximal end to distal end. The important feature is the open slot 6104 to allow any ferrules with size larger than inside diameter of 6101 to get in from both ends and to automatically align the ferrules. This is important to accommodate the variance of the ferrule, as even they are in precise relation to each other.

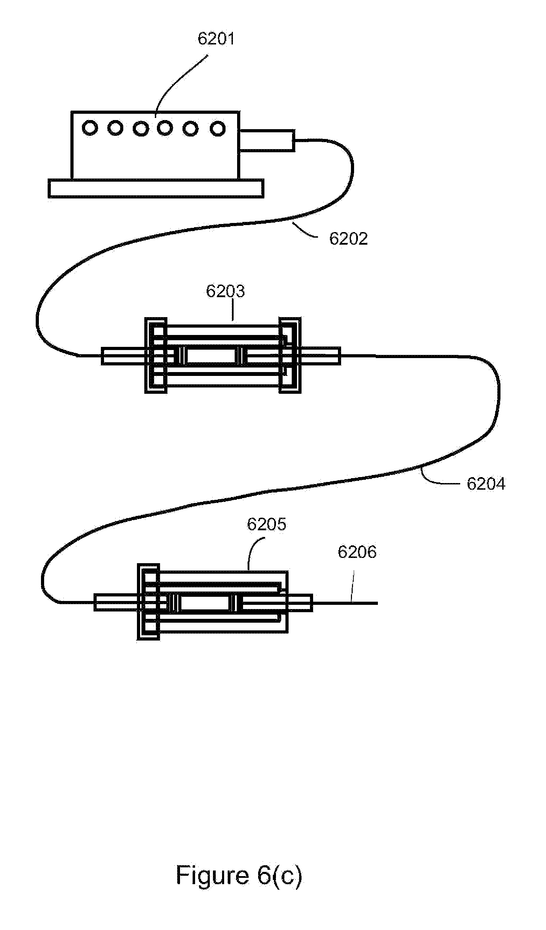

Fig. 6(c) depicts assembled fiber conduction mechanism as layout in Fib.6(a). A laser beam from laser module 6201 is transported through a fiber cable 6202 to a connection point 6203, which contains a coupler, a housing with one lens and spacers, and a ferrule for another fiber 6204. The laser beam is coupled from one fiber to another fiber utilizing connection points 6203. The mechanism of coupler, spacers and lens make the coupling efficiency from one fiber to another fiber to the optimum. The connection 6203 can be the transporting point to transport laser beam from inside the laser system to outside the laser system as depicted in Fig.1 and Fig.2 Then, laser beam is transported to another connection point 6205, contains a coupler, a housing with one lens and spacers, and a ferrule for another fiber 6206, which may deliver the laser beam to the surgical surface. The connection point 6205 can also be the transporting point from the handpiece to the replaceable tip for laser system as depicted in Fig.1 and Fig. 2.

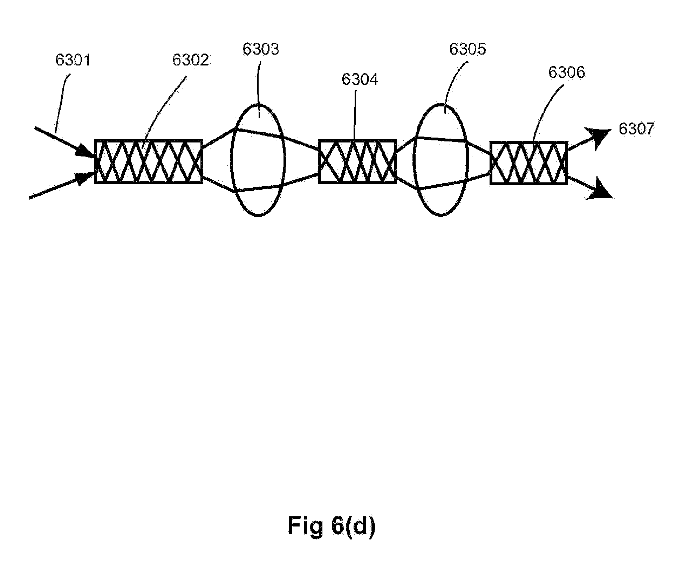

Fig. 6(d) depicts the optical system for laser transportation described in Fig.6(a). A laser beam 6301 inputs to a fiber 6302, then exits from fiber 6302, then focused by lens 6303 to another fiber 6304, then exits fiber 6304, then focused by lens 6305 to another fiber 6306, finally exits at end of 6306 as a beam 6307 to an application surface.

The laser beam deliver mechanism depicted in Fig. 6(d) can be used for a laser system with power output range from 1 to 10 watt.

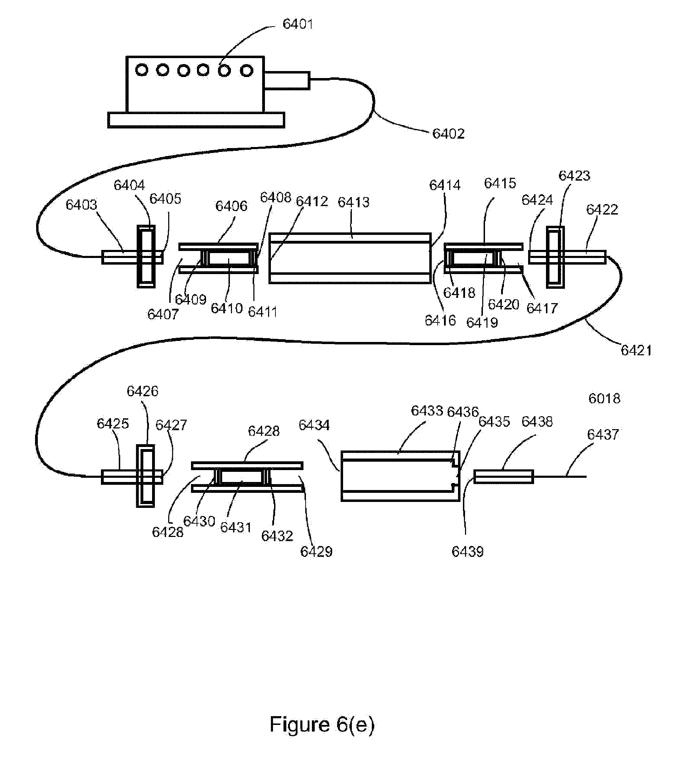

Fig. 6(e) describes another of the delivery mechanisms for a laser beam. Given a laser module 6401 as described above, the system according to the present invention is then assembled with the laser module 6401 as a centerpiece, shown in FIG. 6(e). Fiber 6402 exits module 6401 to connect to other components. A ferrule 6403 is provided to the fiber 6402 so as to connect the fiber 6402 to the next stage. A nut 6404 connected to ferrule 6403 facilitates the connection of ferrule 6403 to other connections. The fiber 6402 is finished at end of ferrule 6405. Then, there is a housing 6406 with an opening 6407 at proximal end and another opening 6408 at distal end. There is a precision spacer 6409, an optical lens 6410, and another precision spacer 6411 inside housing 6406. The housing 6406 is identical to housing 6007 described in Fig. 6(b). A coupler 6413 is provided for further light transportation. The coupler 6413 with opening 6412 at proximal end opening 6414 at distal end. While housing 6406 is inserted within coupler 6413 at proximal end 6412, an identical housing 6415 is likewise inserted into coupler distal end 6414. The structure inside housing 6415 mirrors the structure in housing 6406 in that it contains a precision spacer 6418, an optical lens 6419, and another precision spacer 6420 inside housing 6415. Housing 6415 also presents proximal opening 6416 and distal opening 6417. Distal opening 6417 receives a ferrule 6422 containing fiber 6421, which is finished at the end of ferrule 6424. Ferrule 6422 likewise is attached to a nut 6423 to facilitate connection. This is the first part of connection fiber 6421, which has an identical structure at its other end, specifically there is a ferrule 6425 to make fiber to connect to next stage. A nut 6426 is attached to ferrule 6425 and a fiber finish surface 6427 at end of ferrule 6425. Another housing 6428 with opening 6429 at proximal end and opening 6430 at distal end contains a precision spacer 6431, an lens 6432, and a precision spacer 6433. A coupler 6434 with opening 6435 at proximal end, opening 6436 at distal end, and a stop point 6437 to house 6428. Another ferrule 6439 to contain fiber 6438 with fiber finish 6440 can be fit to coupler 6434. This construction had the added utility of an extra focusing lens over the first embodiment described in FIG 6(a).

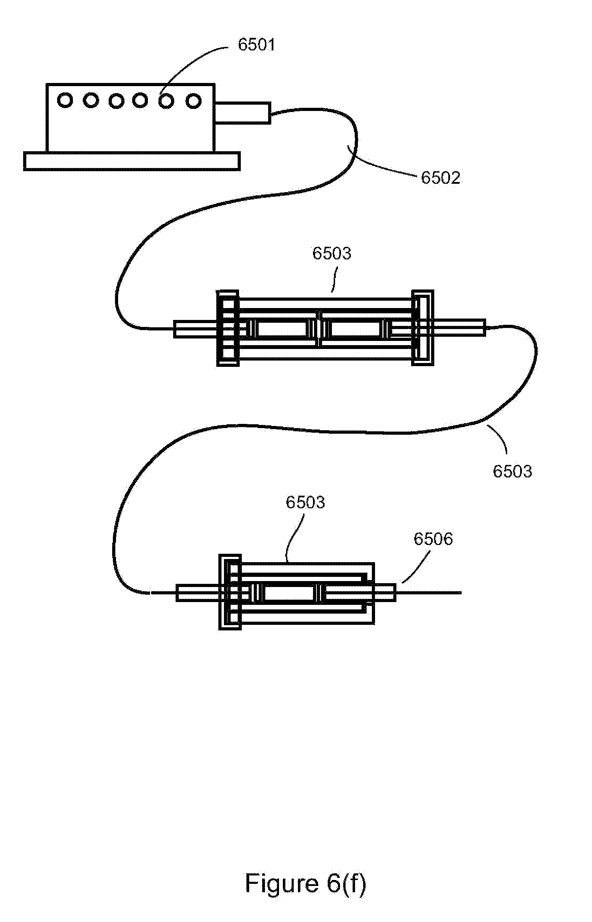

Fig. 6(f) depicts assembled fiber conduction mechanism as layout in Fib.6(e). A laser beam from laser module 6501 is transported through a fiber cable 6502 to a connection point 6503, which contains a coupler, a housing with two lenses, and a ferrule for another fiber 6504. The connection point 6503 can also be the transporting point to transport laser beam from inside the system to the outside the system as depicted in Fig.1 and Fig. 2. Then, laser beam is transported to another connection point 6505, contains a coupler, a housing with one lens, and a ferrule for another fiber 6506, which may deliver the laser beam to the surgical surface. The connection point 6506 can be the transporting point from the handpiece to the replaceable tip for laser system as depicted in Fig.1 and Fig. 2.

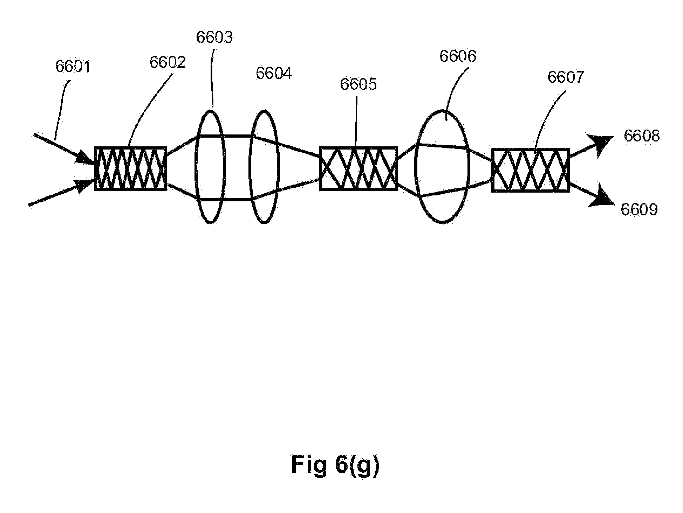

Fig. 6(g) depicts the optical system for laser transportation described in Fig.6(e). A laser beam 6601 inputs to a fiber 6602, then exits from fiber 6602, then focused by lenses 6603 and 6604 to another fiber 6605, then exits fiber 6605, then focused by lens 6606 to another fiber 6607, finally exits at end of 6607 as a beam 6608 to an application surface.

The laser beam deliver system depicted in Fig. 6(g) can be used for a laser system with moderate power output, for example, the final laser output is ranged from 1 to 15 watt.

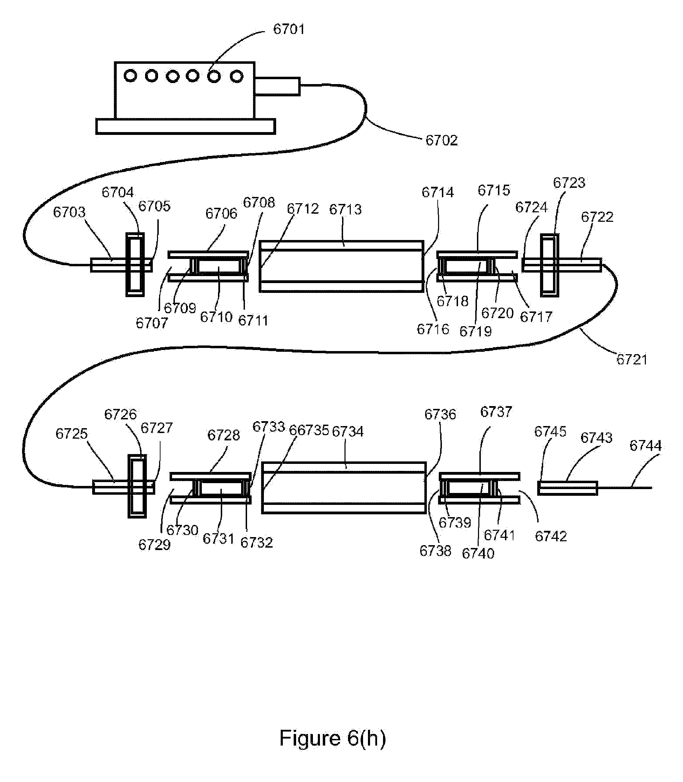

Fig. 6(h) describes another of the delivery mechanisms for a laser beam. Given a laser module 6701 as described above, the system according to the present invention is then assembled with the laser module 6701 as a centerpiece, shown in FIG. 6(h). Fiber 6702 exits module 6701 to connect to other components. A ferrule 6703 is provided to the fiber 6702 so as to connect the fiber 6702 to the next stage. A nut 6704 connected to ferrule 6703 facilitates the connection of ferrule 6703 to other connections. The fiber 6702 is finished at end of ferrule 6705. Then, there is a housing 6706 with an opening 6707 at proximal end and another opening 6708 at distal end. There is a precision spacer 6709, an optical lens 6710, and another precision spacer 6711 inside housing 6706. The housing 6706 is identical to housing 6007 described in Fig. 6(b). A coupler 6713 is provided for further light transportation. The coupler 6713 with opening 6712 at proximal end opening 6714 at distal end. While housing 6706 is inserted within coupler 6713 at proximal end 6712, a housing 6715 is likewise inserted into coupler distal end 6714. The structure inside housing 6715 mirrors the structure in housing 6706 in that it contains a precision spacer 6718, an optical lens 6719, and another precision spacer 2720 inside housing 6715. Housing 6715 also presents proximal opening 6716 and distal opening 6717. Distal opening 6717 receives a ferrule 6722 containing fiber 6721, which is finished at the end of ferrule 6724. Ferrule 6722 likewise is attached to a nut 6723 to facilitate connection. This is the first part of connection fiber 6721, which has an identical structure at its other end, specifically there is a ferrule 6725 to make fiber to connect to next stage. A nut 6726 is attached to ferrule 6725 and a fiber finish surface 6727 at end of ferrule 6725. Another housing 6728 with opening 6729 at proximal end and opening 6733 at distal end contains a precision spacer 6430, an lens 6431, and a precision spacer 6432. A coupler 6734 is provided for further light transportation. The coupler 6734 with opening 6735 at proximal end opening 6736 at distal end. While housing 6728 is inserted within coupler 6734 at proximal end 6735, an identical housing 6737 is likewise inserted into coupler distal end 6736. The structure inside housing 6737 mirrors the structure in housing 6728 in that it contains a precision spacer 6738, an optical lens 6740, and another precision spacer 6741 inside housing 6737. Housing 6737 also presents proximal opening 6738 and distal opening 6742. Distal opening 6742 receives a ferrule 6743 containing fiber 6744, which is finished at the end of ferrule 6745. This construction had the added utility of an two extra focusing lenses over the first embodiment described in FIG 6(a).

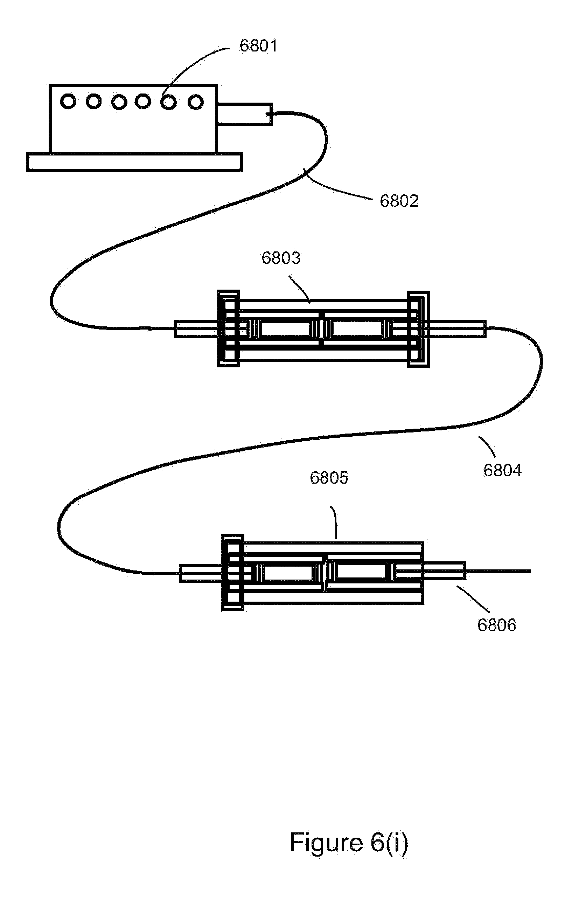

Fig. 6(i) depicts assembled fiber conduction mechanism as layout in Fib.6(h). A laser beam from laser module 6801 is transported through a fiber cable 6802 to a connection point 6803, which contains a coupler, a housing with two lenses, spacers between lens and fiber finishes, and a ferrule for another fiber 6804. Then, laser beam is transported to another connection point 6805, contains a coupler, a housing with two lenses, and a ferrule for another fiber 6806.

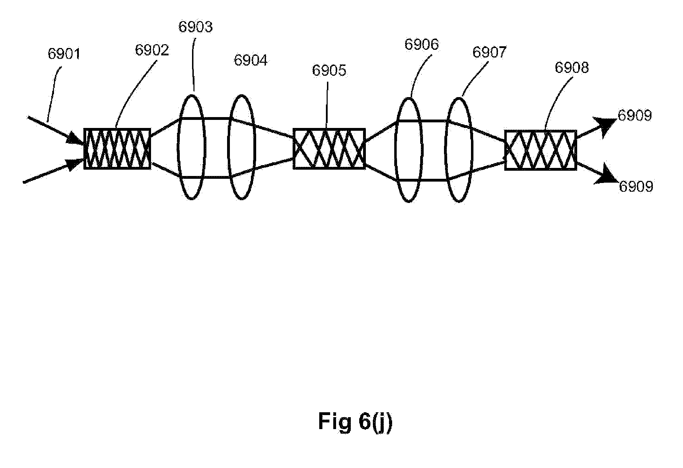

Fig. 6(j) depicts the optical system for laser transportation described in Fig.6(h). A laser beam 6901 inputs to a fiber 6902, then exits from fiber 6902, then focused by lenses 6903 and 6904 to another fiber 6905, then exits fiber 6905, then focused by lenses 6906 and 6907 to another fiber 6608, finally exits at end of 6608 as a beam 6609 to an application surface. The mechanism designed in Fig.6(j) can be useful for high power laser delivery.

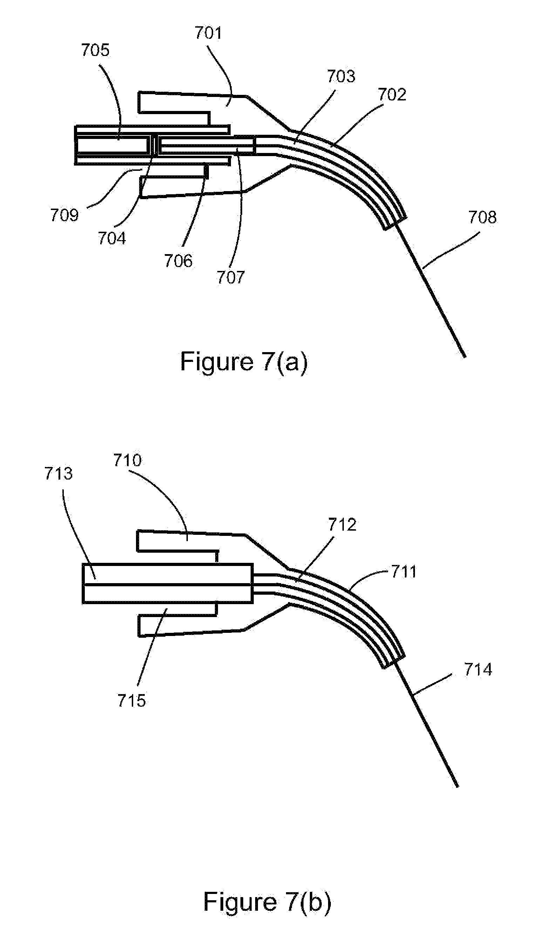

Due to the fiber coupling design in Figures 6(a) -6(j), the fiber tips for surgical purpose can be changed at any given time. A tip design with a housing and an optical lens is illustrated in Figure 7a. The tip comprises a casing 701 from which cannular tip 702 extends. In the cannular tip 702, there is a channel 703 to guide fiber 708. A cylindrical housing 706 contains an optical lens 705, a spacer 704 and a fiber connector 707 which encompasses one end of fiber 708. The fiber 708 will be bent according to the shape of channel 703 which can be straight or any angle. There is an open space 709 so that the tip can fit to the designated handpeice.

The tip shown in FIG. 7b is a tip without an optical lens. Tip comprises casing 710 from which cannular tip 711 extends. In cannular tip 711, there is a channel 71 2 to guide fiber 714. There is a connector 71 3 encompassing fiber 714 inside tip casing 710. The cannular tip 711 can be any angle by designing the casing so that the fiber can be any angle relative to tip axis. There is a space 715 to have tip to fit into handpiece. In either tip embodiment, the fiber in the tip can be versatile and may emit light in different patterns through the physical structure of the tip, as is known in the art and later discovered, including just at end the tip or in all directions. The structure of the tip is such that the fiber 708, 714 is fixedly encased in the tip, with the intention of being disposable while sacrificing as little material resources as possible. By being fixed in the tip and disposable, do not suffer the same stresses as other prior art fibers and can be gently bent to any angle during assembly with little fear of stresses and strain caused by repeated insertion and removal of fibers into other cannula systems.

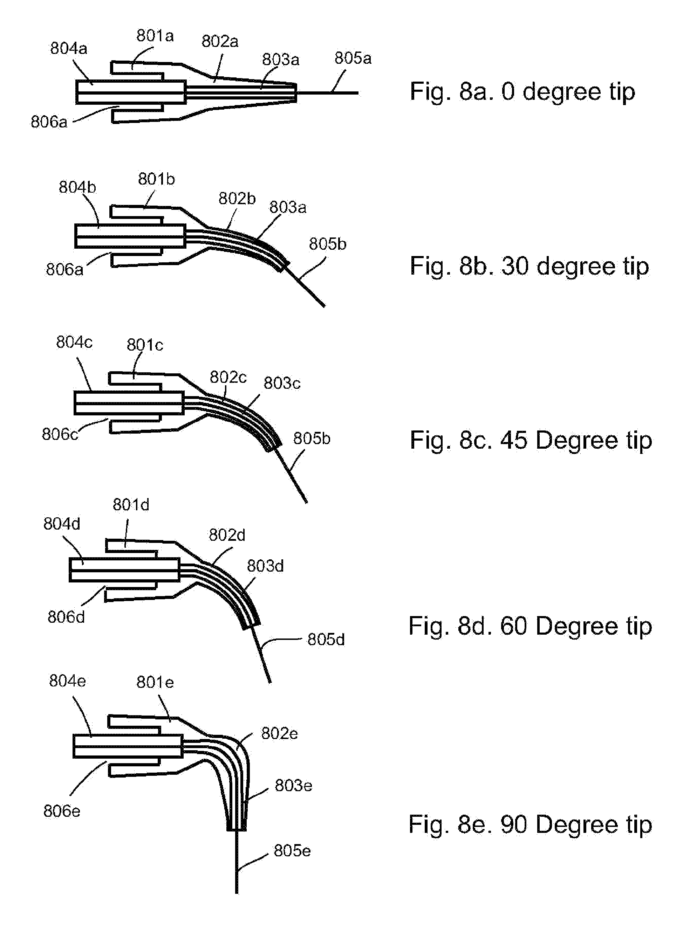

Tips may be offset at any angle from an axis defined by the fiber connectors in the tip. Figures 8a - 8e depict the tip design of FIG. 7b with offsets of 0°, 30°, 45°, 60° and 90° respectively. These angles are of course examples as any angle may be used since casing of each tip supports the fiber and the fiber is not stressed by being repeatedly bent to various degrees when inserted and removed from a cannula or other guide. Each tip has a casing 801 a, 801 b, etc. with a cannular tip 802a, 802b, etc. extending therefrom. Cylindrical connector 804a, 804b, etc encompasses one end of fiber 805a, 805b, etc, and is situated opposite cannular tip 802a, 802b, etc. in the housing 801 a, 801 b, etc. It is surrounded by a space 806a, 806b, etc. to allow for connection to the handpiece. The cylindrical connector 804a, 804b, etc. also defines an axis. Each cannular tip 802a, 802b, etc, contains a channel 803a, 803b etc. and is bent (as is the contained channel 803a, 803b, etc.) to an angle relative to the axis. Fiber 805a, 805b, etc. extends from cylindrical connector 804a, 804b, etc., through channel 803a, 803b, etc. and has its distal end extend out cannular tip 802a, 802b, etc., following the bend in the tip, thereby redirecting the laser received from the connected handpiece.

Although the present invention has been described with reference to preferred embodiments, numerous modifications and variations can be made and still the result will come within the scope of the invention. No limitation with respect to the specific embodiments disclosed herein is intended or should be inferred.

### INDUSTRIAL APPLICABILITY

The invention may be constructed of simple molded plastic, or other suitable material, components, both in the body and the light conducting fiber optic and lens components. Glass and other light conducting materials may be used for the light conducting components. The invention has use in any industry where lasers are used - in particular medical and other fields where tip replacement yields a more hygienic service environment.

## Claims

1. A disposable laser transmission tip for a laser system, the disposable laser transmission tip comprising:
a tip body (701; 710; 801a-e) having a both a distal end and an opposite connection end with a channel (703; 712; 803a-e) extending through the tip body from the connection end through the distal end;
a tip fiber (708; 714; 805a-e) residing within the channel, with first and second ends such that the second end terminates proximate but outside the distal end and the first end terminates proximate the connection end; and
a connection structure (706: 707; 713; 804a-e) coaxially encompassing the first end of the tip fiber, said connection structure fixedly situated at least partially in the connection end;
wherein the connection end of the connection structure is configured to removably attach the tip body to a handpiece (109; 201) and permanently affix the fiber to the tip body, and wherein when the connection end is attached to the handpiece, the connection structure aligns the tip fibre with a fibre of the handpiece to enable laser light to be transported from the fibre of the handpiece of the tip fibre and wherein said connection end of said connection structure and said connection end of said tip body are coaxially arranged to define a space (709; 715; 806a-806e) therebetween for receiving said handpiece therein.

2. The disposable laser transmission tip of claim 1, further comprising an optical lens (705) situated proximate the connection structure and the first end of the tip fiber, a spacing structure (704) between the connection structure and the optical lens, and a connection housing (706) disposed at least partially within the connection end and coaxially encompassing the optical lens, spacing structure and connection structure.

3. The disposable laser transmission tip of claim 2, further comprising the fiber second end being shaped to emit laser light in all directions.

4. The disposable laser transmission tip of claim 2, further comprising the fiber second end being shaped to concentrate laser light emitted at the second end.

5. The disposable laser transmission tip of claim 2, further comprising the distal end of the tip being bent at an angle offset from a longitudinal axis defined by the connection structure and first end of the tip fiber.

6. The disposable laser transmission tip of claim 5, further comprising the fiber second end being shaped to emit laser light in all directions.

7. The disposable laser transmission tip of claim 5, further comprising the fiber second end being shaped to concentrate laser light emitted at the second end.

8. The disposable laser transmission tip of claim 1, further comprising the distal end of the tip being bent at an angle offset from a longitudinal axis defined by the connection structure and first end of the tip fiber.

9. The disposable laser transmission tip of claim 8, further comprising the fiber second end being shaped to emit laser light in all directions.

10. The disposable laser transmission tip of claim 8, further comprising the fiber second end being shaped to concentrate laser light emitted at the second end.

11. The disposable laser transmission tip of claim 1, further comprising the fiber second end being shaped to emit laser light in all directions.

12. The disposable laser transmission tip of claim 1, further comprising the fiber second end being shaped to concentrate laser light emitted at the second end.

## Patentansprüche

1. Einweg-Laserübertragungsspitze für ein Lasersystem, wobei die Einweg-Laserübertragungsspitze Folgendes umfasst:
einen Spitzenkörper (701; 710; 801 a-e) mit einem distalen Ende und einem gegenüber angeordneten Verbindungsende mit einem Kanal (703; 712; 803a-e), der sich durch den Spitzenkörper vom Verbindungsende durch das distale Ende hindurch erstreckt;
eine Spitzenfaser (708; 714; 805a-e), die in dem Kanal angeordnet ist, mit einem ersten und einem zweiten Ende, sodass das zweite Ende nahe dem, aber außerhalb des distalen Endes endet und das erste Ende nahe dem Verbindungsende endet; und
eine Verbindungsanordnung (706; 707; 713; 804a-e), die koaxial das erste Ende der Spitzenfaser umfasst, wobei die Verbindungsanordnung befestigt wenigstens teilweise im Verbindungsende gelagert ist;
wobei das Verbindungsende der Verbindungsanordnung konfiguriert ist, den Spitzenkörper lösbar an einem Handstück (109; 201) zu befestigen und die Faser permanent am Spitzenkörper zu befestigen, und wobei das Verbindungsende an dem Handstück befestigt ist, wobei die Verbindungsanordnung die Spitzenfaser an einer Faser des Handstücks ausrichtet, um zu ermöglichen, dass Laserlicht von der Faser des Handstücks zu der Spitzenfaser transportiert wird und wobei das Verbindungsende der Verbindungsanordnung und das Verbindungsende des Spitzenkörpers koaxial ausgerichtet sind, um einen Raum (709; 715; 806a-806e) dazwischen zu definieren, um das Handstück dazwischen aufzunehmen.

2. Einweg-Laserübertragungsspitze nach Anspruch 1, ferner umfassend eine optische Linse (705), angeordnet nahe der Verbindungsanordnung und dem ersten Ende der Spitzenfaser, eine Abstandhalteranordnung (704) zwischen der Verbindungsanordnung und der optischen Linse, und ein Verbindungsgehäuse (706), wenigstens teilweise innerhalb der Verbindungsendes angeordnet und die optische Linse, die Abstandhalteranordnung und die Verbindungsanordnung koaxial umschließend.

3. Einweg-Laserübertragungsspitze nach Anspruch 2, ferner umfassend, dass das zweite Ende der Faser derart geformt ist, dass es Laserlicht in alle Richtungen ausstrahlt.

4. Einweg-Laserübertragungsspitze nach Anspruch 2, ferner umfassend, dass das zweite Ende der Faser derart geformt ist, dass das an dem zweiten Ende ausgestrahlte Laserlicht gebündelt wird.

5. Einweg-Laserübertragungsspitze nach Anspruch 2, ferner umfassend, dass das distale Ende der Spitze in einem Winkel gebogen ist, der von einer Längsachse absteht, welche durch die Verbindungsanordnung und das erste Ende der Spitzenfaser definiert ist.

6. Einweg-Laserübertragungsspitze nach Anspruch 5, ferner umfassend, dass das zweite Ende der Faser derart geformt ist, dass es Laserlicht in alle Richtungen ausstrahlt.

7. Einweg-Laserübertragungsspitze nach Anspruch 5, ferner umfassend, dass das zweite Ende der Faser derart geformt ist, dass das an dem zweiten Ende ausgestrahlte Laserlicht gebündelt wird.

8. Einweg-Laserübertragungsspitze nach Anspruch 1, ferner umfassend, dass das distale Ende der Spitze in einem Winkel gebogen ist, der von einer Längsachse absteht, welche durch die Verbindungsanordnung und das erste Ende der Spitzenfaser definiert ist.

9. Einweg-Laserübertragungsspitze nach Anspruch 8, ferner umfassend, dass das zweite Ende der Faser derart geformt ist, dass es Laserlicht in alle Richtungen ausstrahlt.

10. Einweg-Laserübertragungsspitze nach Anspruch 8, ferner umfassend, dass das zweite Ende der Faser derart geformt ist, dass das an dem zweiten Ende ausgestrahlte Laserlicht gebündelt wird.

11. Einweg-Laserübertragungsspitze nach Anspruch 1, ferner umfassend, dass das zweite Ende der Faser derart geformt ist, dass es Laserlicht in alle Richtungen ausstrahlt.

12. Einweg-Laserübertragungsspitze nach Anspruch 1, ferner umfassend, dass das zweite Ende der Faser derart geformt ist, dass das an dem zweiten Ende ausgestrahlte Laserlicht gebündelt wird.

## Revendications

1. Embout de transmission laser jetable pour un système laser, l'embout de transmission laser jetable comprenant :
un corps d'embout (701 ; 710 ; 801a-e) ayant à la fois une extrémité distale et une extrémité de connexion opposée avec un canal (703 ; 712 ; 803a-e) s'étendant à travers le corps d'embout depuis l'extrémité de connexion à travers l'extrémité distale ;
une fibre d'embout (708 ; 714 ; 805a-e) résidant dans le canal, avec une première et une seconde extrémité telles que la seconde extrémité se termine à proximité, mais à l'extérieur de l'extrémité distale et que la première extrémité se termine à proximité de l'extrémité de connexion ; et
une structure de connexion (706 ; 707 ; 713 ; 804a-e) enserrant coaxialement la première extrémité de la fibre d'embout, ladite structure de connexion étant située de manière fixe au moins en partie dans l'extrémité de connexion ;
dans lequel l'extrémité de connexion de la structure de connexion est configurée pour fixer de manière amovible le corps d'embout à une pièce à main (109 ; 201) et fixer en permanence la fibre au corps d'embout, et dans lequel, lorsque l'extrémité de connexion est fixée à la pièce à main, la structure de connexion aligne la fibre d'embout à une fibre de la pièce à main pour permettre de transporter la lumière du laser de la fibre de la pièce à main à la fibre d'embout et dans lequel ladite extrémité de connexion de ladite structure de connexion et ladite extrémité de connexion dudit corps d'embout sont aménagées coaxialement pour définir un espace (709 ; 715 ; 806a-806e) entre elles afin d'y recevoir ladite pièce à main.

2. Embout de transmission laser jetable selon la revendication 1, comprenant en outre une lentille optique (705) située à proximité de la structure de connexion et de la première extrémité de la fibre d'embout, une structure d'espacement (704) entre la structure de connexion et la lentille optique et un boîtier de connexion (706) disposé au moins en partie dans l'extrémité de connexion et enserrant coaxialement la lentille optique, la structure d'espacement et la structure de connexion.

3. Embout de transmission laser jetable selon la revendication 2, comprenant en outre la seconde extrémité de la fibre qui est conformée pour émettre de la lumière laser dans toutes les directions.

4. Embout de transmission laser jetable selon la revendication 2, comprenant en outre la seconde extrémité de la fibre qui est conformée pour concentrer la lumière laser émise à la seconde extrémité.

5. Embout de transmission laser jetable selon la revendication 2, comprenant en outre l'extrémité distale de l'embout qui est cintrée selon un angle décalé d'un axe longitudinal défini par la structure de connexion et la première extrémité de la fibre d'embout.

6. Embout de transmission laser jetable selon la revendication 5, comprenant en outre la seconde extrémité de la fibre qui est conformée pour émettre de la lumière laser dans toutes les directions.

7. Embout de transmission laser jetable selon la revendication 5, comprenant en outre la seconde extrémité de la fibre qui est conformée pour concentrer la lumière laser émise à la seconde extrémité.

8. Embout de transmission laser jetable selon la revendication 1, comprenant en outre l'extrémité distale de l'embout qui est cintrée selon un angle décalé d'un axe longitudinal défini par la structure de connexion et la première extrémité de la fibre d'embout.

9. Embout de transmission laser jetable selon la revendication 8, comprenant en outre la seconde extrémité de la fibre qui est conformée pour émettre de la lumière laser dans toutes les directions.

10. Embout de transmission laser jetable selon la revendication 8, comprenant en outre la seconde extrémité de la fibre qui est conformée pour concentrer la lumière laser émise à la seconde extrémité.

11. Embout de transmission laser jetable selon la revendication 1, comprenant en outre la seconde extrémité de la fibre qui est conformée pour émettre de la lumière laser dans toutes les directions.

12. Embout de transmission laser jetable selon la revendication 1, comprenant en outre la seconde extrémité de la fibre qui est conformée pour concentrer la lumière laser émise à la seconde extrémité.
